# EUROPEAN PATENT APPLICATION

(11) **EP 1 114 859 A1**
(43) Date of publication of application: **11.07.2001**
(21) Application number: 99938604.8
(22) Date of filing: 24.08.1999
(51) Int. Cl.: C12N 15/00

(54) **IMMOBILIZED cDNA LIBRARIES**

(30) Priority: 17.09.1998 JP 26294198
(71) Applicant: Helix Research Institute, Kisarazu-shi, Chiba 292-0812 (JP)
(72) Inventor: OTA, Toshio, Fujisawa-shi, Kanagawa 251-0042 (JP); MITSUHASHI, Masato, Irvine, CA 92604 (US); ISOGAI, Takao, Kisarazu-shi, Chiba 292-0833 (JP); WAKAMATSU, Ai, Kisarazu-shi, Chiba 292-0014 (JP)
(74) Representative: VOSSIUS & PARTNER
(86) International application number: JP9904549
(87) International publication number: WO0017335

(57) **Abstract**

A cDNA library in which sense strand cDNAs are immobilized at the 5'-side is provided. A known nucleotide sequence is artificially added to the 3'-side of an antisense strand cDNA (the first strand) and the 5'-side of the second strand is immobilized by using a primer complementary to the above nucleotide sequence. Thus, a cDNA library with excellent qualities, which contain the full-length cDNA at a high possibility, can be obtained.

## Description

### Technical Field

The present invention relates to a cDNA library, a method for synthesizing the library, and a method for preparing an RNA or an RNA library, moreover a protein library in which the cDNA library is a template.

### Background Art

A method using a cDNA reverse-transcribed from mRNA as a template has been applied for a long time as one of approaches in molecular genetics. The use of cDNA enables understanding of the condition of a gene actually expressing in a cell, thus, is an important method for researches as well as an approach using genome, which is genetic information itself, as a material.

In the case of using cDNA as a research material, a cDNA library synthesized based on mRNA is prepared in general. A cDNA library must fulfill the conditions in which a condition of mRNA is reflected as precisely as possible, and the following cloning and screening are easy to proceed. Reflection of a condition in mRNA means maintaining a population of mRNA in original cells. For example, loosing a weakly expressed gene at the cDNA synthesis or at replication of a library prevents efficient researches. Moreover, a quality of a library depends on whether the full-length mRNA is precisely reflected at the extraction of the template RNA or at the cDNA synthesis. Cleavage of a gene can cause a serious obstacle, especially in the screening by using a protein expressed from a gene as an indicator. On the other hand, an easiness of handling in cloning or screening is characterized by, for example, a convenience for insertion into a cloning vector, or a capability of rapidly expressing as certain amount of a protein.

Some representative methods for synthesizing a cDNA library are widely used. Synthesis of the first strand cDNA with a reverse transcriptase using a repetitive part of adenines (A) called poly (A) commonly present at the 3'-side of mRNA is general. Various contrivances have been attempted in cloning methods after the first strand synthesis. In general, while a double strand is produced by synthesizing the second strand using the first strand as a template, a restriction enzyme site at a terminal of cDNA is added by a given means and a vector library is obtained by inserting the cDNA into an appropriate vector. Specifically, the first strand amplified by an oligo dT primer is double-stranded by the Gubler-Hoffman method or by random primers, the 5'-terminal is blunt-ended, and an adapter is ligated to the blunt-ended 5'-terminal. This is treated with a restriction enzyme and inserted into a cloning site in a vector to prepare a vector library. As a vector, a phage vector such as λgt11, and a plasmid vector such as blue script (a product name) are used.

In the above methods, however, there is a problem that 5'-side of mRNA is not always synthesized as a complete cDNA. For example, in the preparation of a double strand using random primers, a short sequence biased to 3' poly (A)-side of an original mRNA tends to be produced. In the Gubler-Hoffman method, a nick is introduced into a template mRNA hybridizing to the first strand cDNA by RNaseH and the second strand cDNA is synthesized using the nick as a replication origin. It is said that a relatively long strand cDNA is easy to obtain by this method.

In addition, as a method for more efficiently obtaining a vector library containing full-length cDNAs, the Okayama-Berg method, in which a C tailing is added to the 5'-terminal using the terminal transferase for directly inserting to a vector (Okayama, H. and Berg, P., High-efficiency cloning of full-length cDNAs., Mol. Cell Biol., 1982, 2, 161-170), is known. An attempt to obtain a full-length cDNA by specifically introducing a synthesized oligonucleotide into the 5'-side of mRNA and synthesizing a double strand cDNA using a primer complementary to this part (Maruyama, S. and Sugano, S., Oligo-capping: A simple method to replace the CAP structure of eukaryotic mRNAs with oligonucleotides., Gene, 1994, 138, 171-174; Merenkova, N. et al., Method for the specific coupling of the CAP of the extremity 5' of a fragment mRNA and preparation of complete cDNA., PCT/FR96/00651, 1996) has been reported. By using these methods, a CAP structure present at the 5'-side terminal in mRNA is specifically replaced by an artificial oligonucleotide. A cDNA containing a sequence in the 5'-terminal region of mRNA can be theoretically obtained by using a sequence complementary to this oligonucleotide as a replication origin for the second strand cDNA synthesis. The number of full-length cDNAs contained in a primary library obtained by such methods is, however, small, and it was difficult to amplify a full-length cDNA library as a master library while maintaining the diversity as a cDNA library.

A cDNA vector library can be obtained from mRNA by the above methods. In addition, in some cases, a cDNA has been immobilized. In the immobilized cDNA library method, an immobilized oligo dT primer method, in which an oligo dT primer immobilized on a solid phase is used (Mitsuhashi, M., Gene manipulation on plastic plates., Nature, 1992, 357, 519-520), is known. Extraction of RNA required for the other methods is not necessary due to capturing mRNA in a sample by immobilized oligo dT primers. The first strand is synthesized using a captured mRNA as a template with a reverse transcriptase. As the oligo dT primer is immobilized, the first strand synthesized at this time is also immobilized. Specifically, a cDNA library obtained here is a library of an antisense strand cDNA immobilized in the 5' side. Separation from the secondary cDNA library synthesized by PCR is easy by using the obtained first strand as a primary library, and moreover, the first strand can be reused. Solid Phase cDNA Synthesis Kit (Takara, a product name) is a kit in which reagents necessary for the immobilized cDNA library method are packaged.

In a library of an antisense strand (the first strand) cDNA immobilized at the 5'-terminal, however, the inclusion of numerous incomplete cDNAs is a problem. The first strand cDNA synthesized on a solid phase by the immobilized oligo dT primers is theoretically a product of the reverse transcription of a whole mRNA containing poly (A). Many of actual mRNAs selected by the oligo dT, however, contain incomplete length in the 5'-side. In the ordinary conditions, a ratio of full-length mRNAs to the whole mRNAs is low. A ratio of full-length mRNAs varies depending on a kind and a condition of a sample from which an mRNA is derived, or the extraction conditions. However, mRNAs with incomplete length are majority. Therefore, a majority of cDNAs constructing a cDNA library immobilized by immobilized oligo dT primers reflects incomplete sequences. Moreover, even if an mRNA is full-length, a ratio of full-length cDNAs is further low due to the fact that complete synthesis of cDNA to the 5'-terminal does not always occur.

If all mRNAs can be captured, conditions in which a population of mRNAs is reflected can be fulfilled. In addition, by combining with the above methods for obtaining full-length cDNAs, providing an immobilized library rich in full-length cDNAs may be expected. In fact, however, a method for immobilizing the 5'-side of the first strand cDNA (corresponding to the 3'-side of mRNA) can not selectively immobilize full-length cDNAs, and thus, does not lead to increase of full-length cDNAs in a library of immobilized cDNAs.

Moreover, in known immobilized oligo dT methods, a difficulty of obtaining the secondary cDNA library using the immobilized first strand cDNA as a master library is a problem. For example, in the case of synthesizing the second strand by random primers, a library in which a population is biased to short fragments tends to be obtained. Even in the condition of containing full-length cDNAs to some extent by the combination of the oligo CAP method, maintenance of excellent quality in the secondary library (i.e. a variety of full-length cDNAs) can not be expected as a ratio of full-length cDNAs to the immobilized cDNAs is low.

A technology for synthesizing an RNA *in vitro* using a cDNA as a template by arranging a promoter sequence which enables *in vitro* RNA synthesis, for example, sequences of T7 promoter, T3 promoter, and SP6 promoter, upstream of the sense strand cDNA is known. By applying this technology to a library of immobilized cDNA, an mRNA library can be synthesized *in vitro* using RNA polymerase. In the case of immobilizing 5'-side of the fist strand cDNA, however, high efficiency of translation into proteins can not be expected due to the low ratio of cDNAs including a part corresponding to the 5'-side of mRNA (a side containing a translation initiation point) previously described.

### Disclosure of the Invention

An objective of the present invention is to provide an immobilized cDNA library comprising a novel structure. More specifically, an objective of the present invention is to provide a cDNA in which the 5'-terminal side of a sense strand cDNA is immobilized on the solid phase. Furthermore, by using the technique, to provide a cDNA library is another objective of the present invention.

In addition, in a preferred embodiment of the present invention, an objective of the present invention is to provide a library of sense strand cDNA immobilized in the 5'-side of excellent quality, in which a ratio of full-length cDNAs is high and, which is useful as a primary library capable of more faithfully reflecting a population of cDNAs in the secondary library. Moreover, in another embodiment of the present invention, an objective is to provide a sense strand cDNA library which can add a given gene sequence, for example, of an RNA polymerase promoter, to upstream of the sense cDNA.

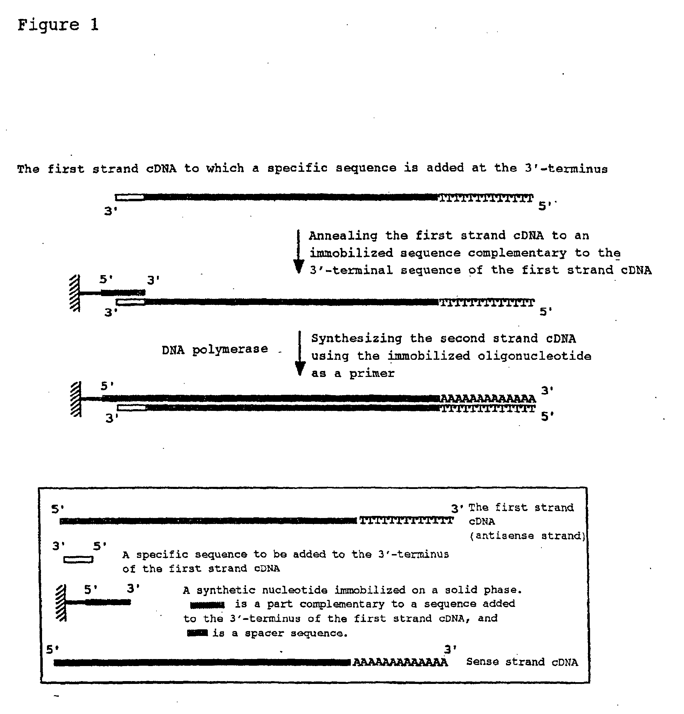

The present inventors supposed that the above objective could be achieved by adding an artificial nucleotide sequence to the 3'-terminal of the first strand cDNA (an antisense strand) and using this nucleotide sequence. More specifically, the first strand cDNA can be captured by previously immobilizing the 5'-terminal side of a synthetic oligonucleotide containing a sequence complementary to this artificial nucleotide sequence in the 3'-terminal side on a solid phase, and hybridizing with this synthetic oligonucleotide. Any cDNA can be then synthesized on the solid phase in the immobilized form of 5'-terminal side of a sense strand by synthesizing the second strand (i.e. a sense strand) cDNA in the direction from 3' to 5' of the first strand by using the immobilized synthetic oligonucleotide as a primer and the captured first strand (an antisense strand) cDNA as a template (Figure 1).

Moreover, the present inventors have found that various effects can be expected by immobilizing the 5'-side of a sense strand (i.e. the second strand). More specifically, for example, the present inventors have completed the present invention by finding that an ideal cDNA library theoretically composed of only full-length cDNAs can be provided by applying a method of synthesizing cDNA according to the present invention to the synthesis of a cDNA library. Alternatively, any nucleotide sequence can be arranged upstream of a sense strand in the immobilized cDNA library by adding a nucleotide sequence to 5'-side of a sense strand, thereby finding a novel use of a cDNA library. More specifically the present invention relates to the following cDNA libraries, methods for preparing the cDNA libraries, and use of the libraries.
(1) A cDNA library in which sense strand cDNAs are immobilized at the 5'-side.
(2) The cDNA library of (1), wherein a common nucleotide sequence to cDNAs constituting the library is present at the 5'-terminal of the sense strand cDNAs.
(3) The cDNA library of (2), wherein the common nucleotide sequence is the sense sequence of a promoter specifically recognized by an RNA polymerase.
(4) The cDNA library of (2), wherein the common nucleotide sequence encodes an arbitrary amino acid sequence and wherein the nucleotide sequence constitutes the same reading frame as the cDNAs.
(5) The cDNA library of (1), wherein the sense strand cDNAs comprise a translation initiation codon.
(6) The cDNA library of (5), wherein the translation initiation codon is derived from an mRNA.
(7) A method for synthesizing a cDNA, wherein a known nucleotide sequence is artificially added to the 3'-terminal of a first strand cDNA and wherein an oligonucleotide used as a primer for synthesizing a second strand binds to a solid phase at the 5'-side, the method comprising:
   a) synthesizing the first strand cDNA using an mRNA as a template with a primer for synthesizing the first strand cDNA, and
   b) synthesizing a sense strand cDNA using, as a primer for synthesizing the second strand, an oligonucleotide comprising a sequence complementary to the 3'-side of the first strand cDNA produced in a).
(8) The method of (7), wherein the known nucleotide sequence is added to the 3'-terminal of the first strand cDNA by:
   a) binding an oligonucleotide comprising a known sequence to the 5'-terminal of an mRNA, and
   b) synthesizing the first strand cDNA using the mRNA of a) as a template with a primer for synthesizing the first strand.
(9) The method of (8), wherein the oligonucleotide is bound in a) above by a method in which a CAP structure present at the 5'-terminal of the mRNA is specifically recognized.
(10) A sense strand cDNA immobilized at the 5'-side, the sense strand cDNA which can be obtained by the method of any one of (7) to (9).
(11) A method for synthesizing a cDNA library by the method of any one of (7) to (9) using an mRNA as a starting material.
(12) A cDNA library in which sense strand cDNAs are immobilized at the 5'-side, the cDNA library which can be obtained by the method of (11).
(13) A cDNA library comprising full-length cDNAs, the cDNA library which can be obtained by the method of (9) using an mRNA as a starting material.
(14) A secondary cDNA library which can be obtained by amplifying the cDNA library of (12) or (13).
(15) A method for obtaining an mRNA library, the method comprising synthesizing RNAs using the cDNA library of (3) as a template with a DNA-dependent RNA polymerase recognizing the promoter of (3).
(16) An mRNA library which can be obtained by the method of (15).
(17) A method for preparing a protein library, the method comprising translating the mRNA library of (16) into proteins with an expression system.
(18) A protein library which can be obtained by the method of (17).
(19) A method for subtracting cDNAs, the method comprising:
   a) synthesizing cDNAs used as testers,
   b) hybridizing the cDNAs using the sense strand cDNA library of any one of (1), (12), and (13) as a driver, and
   c) selecting cDNAs which have or have not hybridized in b).

By the present invention, a sense strand cDNA immobilized at the 5'-side is provided. The immobilized cDNA can be either single or double strand. In the case of a double strand, the strand may be double stranded in full length or in a part. Any strand can be used as long as it is capable of reconstructing a double strand by a reaction for synthesizing a complementary strand.

In the present invention, a sense strand means a sequence maintaining genetic information. Specifically, a nucleotide sequence of an mRNA is a sense strand. In contrast, an antisense strand means a nucleotide sequence complementary to the sense strand. Therefore, the first strand cDNA synthesized by using mRNA as a template comprises an antisense sequence. A cDNA library of the present invention is a complex of DNAs synthesized by using, as a template, an mRNA whose sequence is unknown (i.e. cDNAs). In the present invention, an unknown sequence simply means a sequence in which an individual RNA sequence is not specified. Thus, known and unknown sequences are mixed. On the other hand, cDNA solely described herein means a cDNA obtained by using a specific mRNA as a template. Moreover, the immobilization of 5'-side includes the immobilization not only at 5'-terminal but also close to 5'-terminal of a sense strand cDNA.

In a preferred embodiment, a cDNA library is required to reflect an mRNA population as faithfully as possible, but it may comprise a bias depending on a purpose. Alternatively, a library in the intentionally biased condition may be occasionally required.

In the present invention, a known nucleotide sequence artificially added to 3'-terminal of a first strand cDNA can be any sequence as long as it is able to hybridize with an oligonucleotide comprising the complementary sequence. A sense strand cDNA is synthesized by priming from the above artificially added known nucleotide sequence part. At this time, the second strand cDNA synthesized is immobilized by immobilizing the 5'-terminal of a primer on a solid phase. As a method for immobilizing an oligonucleotide on a solid phase, some chemical methods are known.

In a preferable embodiment of the present invention, as the above artificially added known nucleotide sequence, a functional gene sequence, for example, an antisense sequence of a promoter recognized by an RNA polymerase or an antisense sequence for a given protein, can be selected. When these sequences are used, a synthesized sense strand cDNA library finally comprises the structure which arranges a functional sequence upstream of the sense strand cDNA. Moreover, in the present invention, by immobilizing a sense strand at the 5'-side, a ratio of full-length cDNAs can be increased, and a cDNA library comprising a target structure can be easily prepared even when a sequence arranged upstream of a sense strand cDNA is relatively long. As a result, in an RNA transcribed based on this cDNA library, a region comprising a translation initiation point at the 5'-side in mRNA used as a source is reconstructed with the high probability. An RNA comprising a translation initiation point can be translated into a protein. A cDNA library comprising this kind of structure is a novel, and thus some application technologies provided by this structure are also novel. Some characteristic structures obtained by the present invention and an applied technology of a novel cDNA library achieved by this structure are more specifically described later.

The present invention also provides a method for synthesizing the above sense strand cDNA immobilized at the 5'-side. In a preferred embodiment of a method for synthesizing cDNAs based on the present invention, a novel immobilized cDNA library comprising numerous full-length cDNAs can be provided. A method for synthesizing such an immobilized cDNA library of the present invention is illustrated in detail below.

A cDNA of the present invention is immobilized at the 5'-terminal of the sense strand on a solid phase. A cDNA of such a structure can be obtained by artificially adding a known nucleotide sequence to the 3'-terminal of a first strand cDNA, while by immobilizing a primer comprising a sequence complementary to the above artificially added known nucleotide sequence when synthesizing the second strand (i.e. a sense strand) using the first strand cDNA as template by using the above primer. Immobilization of a primer can be achieved by, for example, previously immobilizing it on an appropriate solid phase. As a carrier for immobilization, for example, a microtiter plate, a plastic tube, or micro beads can be used depending on the use. A carrier in a microparticle form is an especially desirable material because merits, for example, a broad reaction area and a capability of separation with a magnet in the case that the carrier is a magnetic one, can be expected. As a method for immobilizing an oligonucleotide on a solid phase, for example, a method in which 5'-terminal of an oligonucleotide is covalently bound to a plate using a cross linker (US. Patent No. 5,656,462) is known. Alternatively, by introducing a molecule comprising a binding affinity, for example, biotin, to a base at or close to the 5'-terminal, immobilization not only at the terminal part, but also close to the 5'-terminal is possible by binding it to a solid-phased avidin. A molecule with a binding affinity can be introduced into any site as long as it is capable of functioning as a primer.

In the present invention, a cDNA library can be obtained by using the unspecified number of mRNAs (i.e. an mRNA library) as mRNAs for a starting material of cDNA. An mRNA library can be obtained by a known method using, for example, cultured cells or tissues as a material. More specifically, for example, guanidine thiocyanate-cesium chloride method (Molecular Cloning 2nd Ed., p7. 10, 1989), guanidine thiocyanate-cesium trifluoroacetate method (H. Okayama et al., Methods in Enzymology., 1987, 154, 3) are known. A kit in which reagents necessary for these methods are packaged (RNA Extraction Kit; manufactured by Pharmacia, a product name) is provided in the market. A cDNA of the present invention can be obtained by using not only an eukaryotic mRNA, but also a prokaryotic mRNA and genome of an RNA virus as a template. These RNAs, different from eukaryotes, may not comprise a poly (A) structure. Therefore, random primers and such are necessary to use for synthesizing a first strand.

On the other hand, in the present invention, as a method for artificially adding a known nucleotide sequence to the 3'-terminal of a first strand cDNA, for example, a method in which a known nucleotide sequence is directly and artificially added to the 3'-terminal of the first strand cDNA after the synthesis of the first strand cDNA, or a method in which a sequence complementary to the above known nucleotide sequence has been previously added to the 5'-terminal of an mRNA and a fist strand cDNA comprising the known nucleotide sequence at the 3-terminal is synthesized by using the above mRNA as a template, can be used. Each method which can be used for synthesizing a sense strand cDNA of the present invention is illustrated below. Description is made in the following order, corresponding to a step for synthesis.
1: The artificial addition of a nucleotide sequence to the 3'-terminal of a first strand cDNA
2: Variations for introducing the nucleotide sequence specifically to a full-length mRNA
3: Synthesis of a first strand cDNA (an antisense strand)
4: Synthesis of a second strand cDNA (a sense strand)

### [A method for artificially adding a known nucleotide sequence to the 3'-terminal of a first strand cDNA]

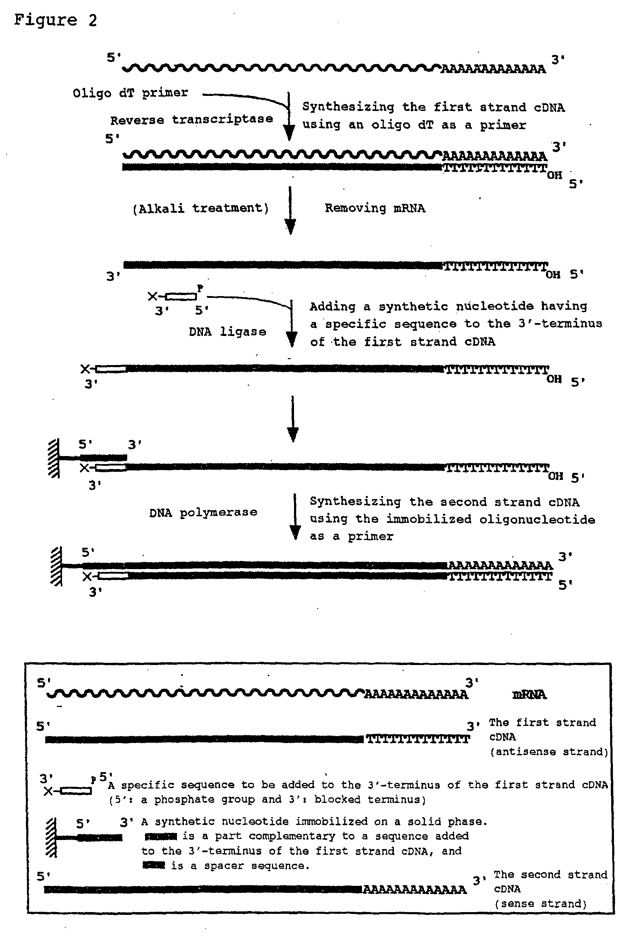

As a method for artificially adding an known nucleotide sequence to the 3'-terminal of a first strand cDNA, for example, the known nucleotide sequence can be directly added to the 3'-terminal of the first strand cDNA after the synthesis of the first strand cDNA (Figure 2). First, an oligonucleotide comprising a given sequence (an nucleotide sequence to be added) which comprises a phosphate group at the 5'-terminal and is structurally blocked not to cause ligation at the 3'-terminal is synthesized. A method for chemically synthesizing an oligonucleotide comprising a given nucleotide sequence is known. On the other hand, a first strand cDNA is synthesized using, as a primer, an oligo dT primer or an oligo dT adapter in which the 5'-terminal is not phosphorylated. Specific ligation occurs between a phosphate group at the 5'-terminal of the synthetic oligonucleotide and a hydroxyl group at the 3'-terminal of the first strand cDNA in the ligation reaction, and the above synthetic oligonucleotide is bound to the 3'-terminal of the first strand cDNA. As the above synthetic oligonucleotide sequence is known, a known nucleotide sequence is finally added to the 3'-terminal of the first strand cDNA.

For blocking the 3'-terminal of the above synthetic oligonucleotide, for example, residues at the 3'-terminal can be dideoxynucleotides. Alternatively, a hydroxyl group at the 3'-terminal can be modified into, for example, an amide group.

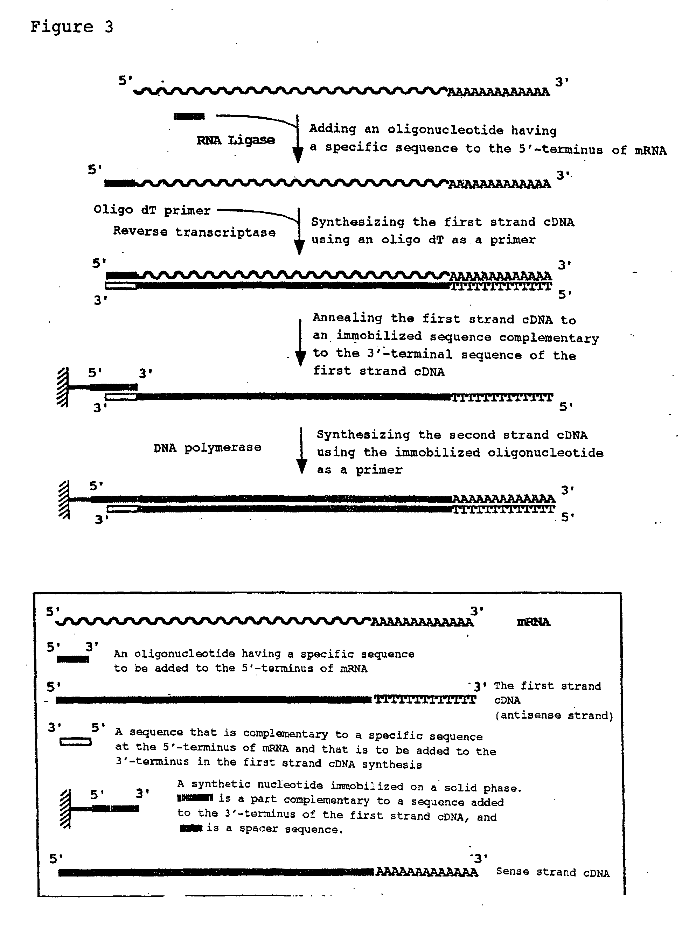

Against the method for modifying the first strand cDNA after the synthesis, a method for modifying an mRNA as a template can be adopted. More specifically, a sequence complementary to a known nucleotide sequence to be artificially added has been previously added to the 5'-terminal of an mRNA. Using this mRNA as a template, the first strand cDNA is synthesized, resulting in the addition of a complementary sequence to the sequence added to 5'-terminal of the mRNA (i.e. the known nucleotide sequence) to the 3'-terminal (Figure 3). To artificially add a known nucleotide sequence to the 5'-terminal of an mRNA, for example, a method in which a synthetic oligonucleotide is added to the 5'-phosphate group terminal of an mRNA molecule using an RNA ligase can be used. As a synthetic oligonucleotide, for example, a synthetic oligo RNA, a synthetic oligo DNA-RNA hybrid, and a synthetic oligo DNA, can be used. If an RNA is necessary to decompose and remove prior to the second strand synthesis, making a synthetic oligonucleotide RNA so as to be removed together is advantageous. When the 5'-terminal of an mRNA is a hydroxyl group, a synthetic oligonucleotide can be more efficiently added by converting the hydroxyl group to a phosphate group. Phosphorylation can be achieved by, for example, the treatment with a T4 nucleotide kinase.

### [A method for artificially adding a known nucleotide sequence selectively to the 5'-terminal of a full-length cDNA (a first strand)]

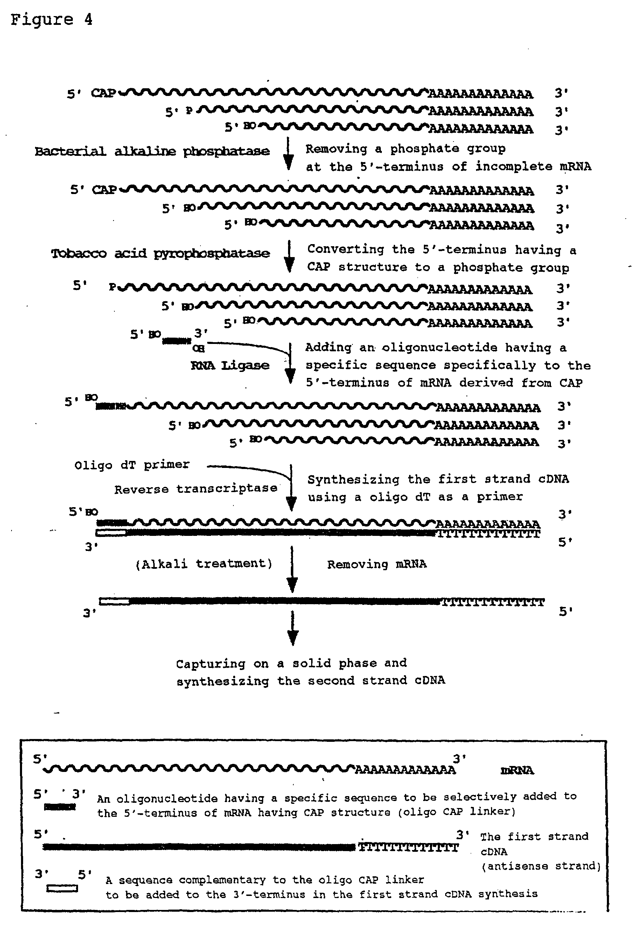

In some variations showed first as specific examples, an artificial nucleotide sequence is added to all mRNAs in any cases. Against these, a method in which an artificial nucleotide sequence can be added only to a full-length mRNA can be adopted. When a source of mRNAs is an eukaryote, a specific structure called the CAP structure is present at the 5'-terminal of a complete mRNA. It is known that by selectively adding a synthetic oligonucleotide against this CAP structure, a cDNA library comprising a translation initiation codon at the high frequency can be finally prepared. By applying this principle to the present invention, a cDNA library containing full-length cDNAs at the higher ratio can be prepared. More specifically, an oligonucleotide comprising a sequence complementary against the above known nucleotide sequence is artificially added specifically to the CAP structure part (Figure 4).

As a method for selectively adding an oligonucleotide against the CAP structure, an oligo-CAP method, in which an mRNA treated with alkaline phosphatase is treated with tobacco acidic phosphatase, then a synthetic oligonucleotide is added thereto using an RNA ligase (Maruyama, S. and Sugano S., Oligo-capping: A simple method to replace the CAP structure of eukaryotic mRNAs with oligonucleotides., Gene, 1994, 138, 171-174), is known. In addition, the modified oligo-CAP method, in which a synthetic DNA-RNA hybrid is used in stead of a synthetic RNA (Kato, S. et al., Gene, 1994, 150, 243-250; Kato and Sekine, Unexamined Published Japanese Patent Application (JP-A) No. Hei 6-153953, June 3, 1994), the linker chemical binding method, in which a diol characteristic in the CAP structure is oxidatively cleaved to convert into an aldehyde group, and chemically bound to the synthetic oligonucleotide to which an amide group has been added at the 3'-terminal (Merenkova, N. and Edwards, D. M., WO 96/34981, Nov. 7, 1996), and so on are known. By using these methods, the CAP structures present at the 5'-side terminal of an mRNA can be specifically replaced by an artificial oligonucleotide. Using the mRNA obtained in this manner as a template, only the full-length first strand cDNA comprise a known nucleotide sequence artificially added to the 3'-side among the first strand cDNAs synthesized by a primer for synthesizing the first strand.

### [A first strand cDNA (an antisense strand)]

Synthesis of a first strand cDNA in the present invention can be achieved by known methods. Depending on embodiments of methods for adding an artificial nucleotide sequence to the 5'-side, the timing differs as previously described. Specifically, the difference is the addition of an artificial nucleotide sequence, at the stage of an mRNA prior to the first strand synthesis or after the synthesis of the first strand cDNA as usual. A method for synthesizing the first strand, applicable to the present invention, is specifically illustrated. A variation of the first strand synthesis depends on the selection of a primer. For synthesizing all mRNAs derived from eukaryotic cells as the first strands, an oligo dT primer is used. In the case of using an RNA of a prokaryotic organism or a virus without poly (A) structure, the use of, for example, a random primer, should be considered.

A general cDNA library is required to reflect a population of mRNAs, or faithfully collect expressed genes. Occasionally, however, manipulation for positively focusing a target gene may be intentionally added. For example, a case in which a library of genes specifically expressed in a specific cellular population is prepared by subtraction is proposed. In another case, for example, for isolating genes in which only a sequence at the 3'-side has been determined, a library composed of only candidate genes similar in the structure of the 3'-side can be prepared by synthesizing the first strand using a sequence of this determined part as a primer. Alternatively, in a gene encoding a variable region of an immunoglobulin, a library of genes in a variable region can be obtained by synthesizing the first strand based on the relatively highly conserved structure at the 3'-side. These primers for synthesizing the first strand are not necessarily completely complementary to the nucleotide sequence of a target mRNA. Synthesis of a complementary strand can be initiated as long as the primer can anneal to the complementary strand under the given stringency and at least the 3'-terminal is completely complementary.

The above primers for synthesizing the first strand can be chemically synthesized. The first strand is synthesized by using an mRNA as a template, by annealing the obtained primer with the mRNA and by reacting reverse transcriptase under the presence of dNTP. The obtained first strand can be used as a template for synthesizing the second strand by various methods by following the variations of methods for artificially adding the above nucleotide sequence.

### Synthesis of a second strand cDNA

In the present invention, a second strand can be synthesized using a sequence complementary (a sense sequence) to a known nucleotide sequence artificially added to a first strand cDNA as a primer. The 5'-terminal of the second strand cDNA (a sense strand) is immobilized if a primer for synthesizing the second strand is immobilized at the 5'-side at this time.

In an embodiment of artificially adding a sequence specifically to full-length cDNA as described above, a known nucleotide sequence part artificially added to the 3'-side in the full-length cDNA is specifically annealed, and as a result, the full-length cDNA is specifically immobilized in theory. In the present invention, the synthesized second strand (a sense strand cDNA) is rarely free because it is immobilized on a solid phase, and even if loosing a complementary strand, a double strand is easily reconstructed using an oligo dT primer. A library of second strands (a sense strand) obtained in this manner can be used as a library of full-length double strand cDNA comprising a translation initiation codon at the high frequency. Based on such a method, a cDNA library theoretically composed of sole full-length cDNAs can be constructed. In fact, however, the possibility of existence of incomplete length cDNAs to some extent can not be denied. Specifically, a full-length cDNA library in the present invention is not necessarily composed of sole full-length cDNAs, but comprises a library with full-length cDNAs at the high ratio. To quantitatively understand the ratio of full-length cDNA to a cDNA library, for example, a program for estimating a probability of comprising a translation initiation codon by analyzing a nucleotide sequence of cDNA can be used. As this kind of programs, Gene Finder (Solovyev V. V., Salamov A. A., Lawrence C. B., Predicting internal exons by oligonucleotide composition and discriminant analysis of spliceable open reading frames., 1994, Nucleic Acids Res., 22, 5156-63) is known. Alternatively, Japanese Patent Application No. Hei 9-289982 by the present inventors has disclosed the method which can more precisely predict a translation initiation codon.

In general, a part of total mRNAs is estimated to give full-length cDNAs even under the ideal conditions. Presence of incomplete length cDNAs is not always disadvantageous. However, for example, a nucleic acid synthetic reaction such as PCR tends to preferentially synthesize short sequences. The synthesized short sequence may prevent, for example, isolation of full-length cDNAs, thus the maintenance of the low ratio of cDNA with incomplete length is necessary for a cDNA library of excellent quality.

In the above manner, a cDNA of the present invention can be synthesized. By known immobilized oligo dT methods, an immobilized library in which the 5'-side of the first strand (an antisense strand) is immobilized can be prepared. A cDNA library immobilized in this manner is, however, different from a cDNA library of the present invention, and comprises numerous incomplete-length cDNAs. In the following, an artificial nucleotide sequence to be added to the 5'-side of the sense strand is described.

### [Variations of known nucleotide sequences to be artificially added]

As a sequence which is artificially added to upstream of the 5'-terminal of the sense strand cDNA to be immobilized in the present invention, any nucleotide sequence can be adopted. In a basic embodiment, any nucleotide sequences capable of annealing with a primer for synthesizing a second strand (a sense strand) can be used. Moreover, when this nucleotide sequence is a functional sequence, not only the nucleotide sequence anneals with a primer, but also a cDNA library of the present invention can be applied in various forms based on the function. Following is variations for artificial known nucleotide sequences and the application thereof.

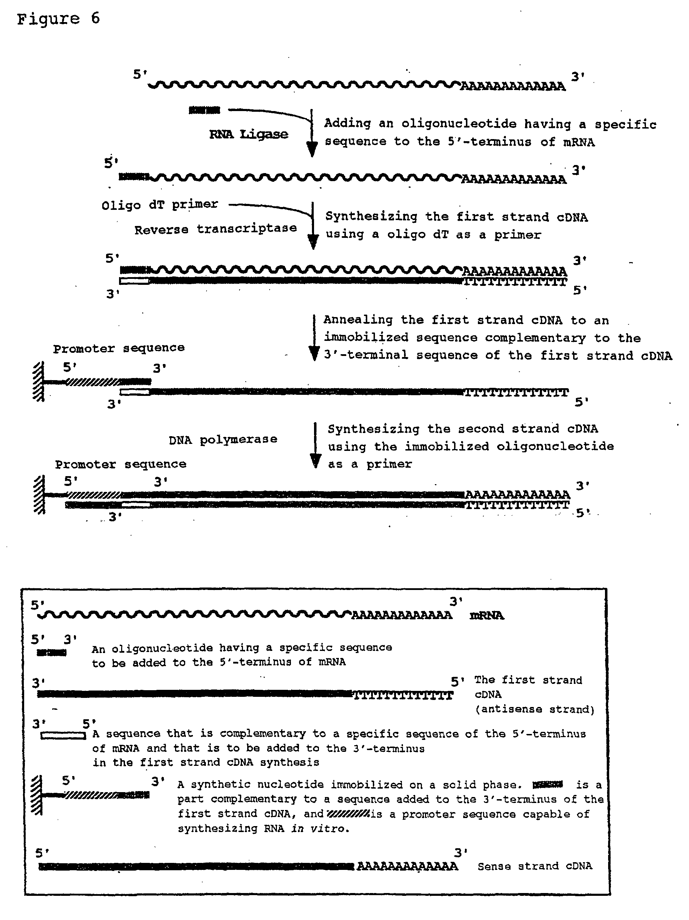

For artificially adding a nucleotide sequence, several embodiments of a method for addition can be illustrated. For example, a sequence can be provided as a sequence added at the 3'-terminal of the antisense strand. A sequence which anneals to this sequence and which becomes a primer for synthesizing the sense strand is a sequence to be artificially added to upstream of the 5' terminal in the sense sequence. A region which extends toward the upstream can be added to this primer. This region extends toward more upstream than the region annealed to the first strand. Thus, the region does not anneal with the first strand but constructs the 5'-side of the second strand. In this case, the 3'-terminal part of an antisense strand anneals to a part of primer. However, the 3'-side of the primer still anneals to the antisense strand, and thus the synthesis of a complementary strand progresses. This sequence projected toward upstream can be kept as a single strand. Alternatively, when a functional nucleotide sequence can not function without forming a double strand (for example, like a promoter sequence in Figure 6), a nucleotide sequence complementary to a functional sequence to be added is synthesized to complete a double strand by further progressing the synthesis of complementary strand in the first strand (an antisense strand) side (described below). In these embodiments, even a long nucleotide sequence can be easily added. A specific region, for example, a region for annealing to the first strand or a region extending toward upstream described here is only for the explanation. Therefore in fact, a part of a functional nucleotide sequence also functions as a region for annealing to the first strand, and a rest part is located upstream thereof.

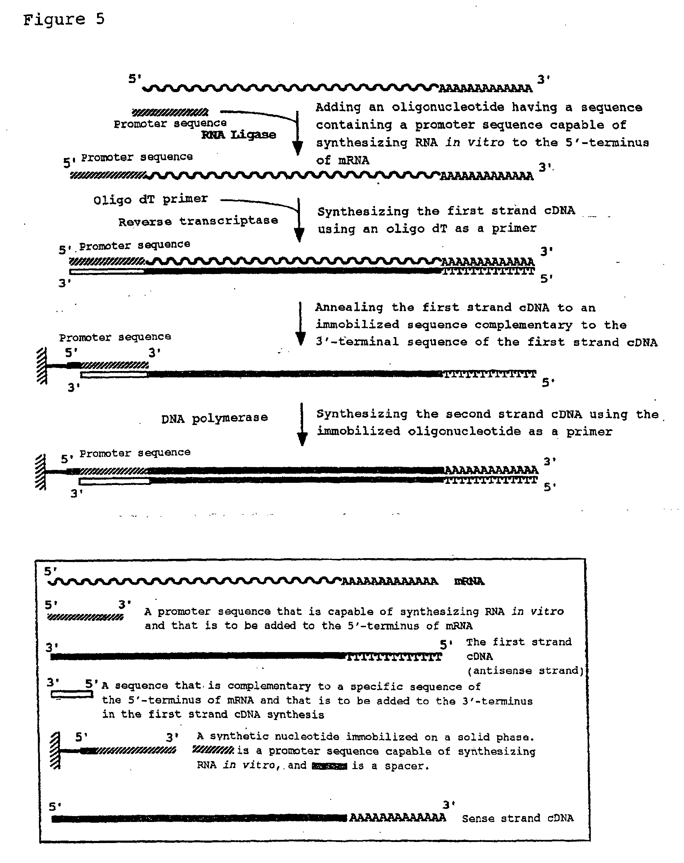

The first variation useful as a known nucleotide sequence to be artificially added is a promoter specifically recognized by an RNA polymerase (Figure 5). A cDNA library encoding fusion proteins by arranging a sense strand which encodes a given protein can be constructed. First, a combination with a promoter will be described.

When a promoter specifically recognized by an RNA polymerase is used as an artificially added known nucleotide sequence in the present invention, the promoter can be arranged upstream of the sense strand cDNA. If this promoter is a promoter sequence specifically recognized by an RNA polymerase capable of synthesizing an RNA *in vitro,* an RNA is transcribed using a cDNA as a template by the RNA polymerase. Examples of a promoter enabling such an application are a T7 promoter sequence (Pribnow, D., Proc. Natl. Acad. Sci. USA., 1975, 72/3, 784-788), a T3 promoter sequence (Adhya, S., Proc. Natl. Acad. Sci. USA., 1981, 78/1, 147-151,), and an SP6 promoter sequence (Brown, J. E., Nucleic Acids Res., 1986, 14/8, 3521-3526). These promoters do not always require a whole sequence, and only a domain necessary for maintaining a promoter activity can be used. Essential sequences (sense strands) of each promoter are as follows. This nucleotide sequence is a sense strand sequence. Thus, to add to the 3'-terminal of the first strand cDNA (an antisense strand), an antisense sequence against the following sequence is used.

T7: TAATACGACTCACTATAGGG (SEQ ID NO: 1)

T3: AATTAACCCTCACTAAAGGG (SEQ ID NO: 2)

SP6: ATTTAGGTGACACTATAG (SEQ ID NO: 3)

In order to transcribe an RNA based on the present invention *in vitro,* the following manipulation is conducted. More specifically, to the cDNA in which a promoter is arranged upstream by the present invention, a ribonucleotide (rNTP) necessary for the synthesis of an RNA is added and RNA polymerase which recognizes the used promoter is reacted. Contamination of the ribonuclease to the reaction solution should be avoided, and more preferably, a ribonuclease inhibitor is added. By reaction for 30 min to several hours, RNAs of µg order can be transcribed. A cDNA used as a template can be easily separated by separating a solid phase after the termination of the transcription. The fact that an isolated cDNA can be easily reused after washing is one of the major characteristics of the present invention. On the other hand, the transcribed RNAs are separated from enzymes and non-reacted substrate rNTPs and collected by extraction with phenol-chloroform and precipitation with ethanol. If cDNAs used as a template are a library, mRNAs can be obtained as a library.

At this time, a cDNA library of the present invention can be directly used as a template, and the secondary cDNA library can be also used as a template. More specifically, the secondary library PCR-amplified using a cDNA library of the present invention as the primary library is used as a template. A primary library means a library which functions as a template for synthesizing a secondary library. While a primary library relatively faithfully reflects an original population of mRNAs, cDNAs per one kind of mRNA are possibly little. Thus, the number of templates for synthesizing RNA is small. Because an ability of transcription in an RNA polymerase is limited, larger amount of transcription products can be expected by increasing the templates. To obtain a secondary library, against a cDNA library of the present invention, PCR is conducted with an oligo dT primer and a primer comprising a sequence complementary to a known nucleotide sequence artificially added to the first strand. By immobilizing any of the primers, a secondary library can be obtained in the immobilized condition. Moreover, if a carrier for immobilizing a primer for synthesizing the secondary library can be separated from a carrier in which the primary library is immobilized, a synthesized secondary library can be easily separated. For example, in the case of immobilizing a primary library on the inner wall of a container, by immobilizing a secondary library on the carrier in a particle form, the both can be easily separated.

A cDNA library which can be obtained by immobilizing the sense strand at the 5'-side according to the present invention contains full-length cDNAs at a high ratio at the stage of a template. Thus, the library is useful for using as a primary library for highly reproducibly obtaining a secondary library in the condition of maintaining a certain diversity as a library. In the present invention, a primary library capable of providing a secondary library in the condition of maintaining such a ratio of full-length cDNAs is specifically called a master library.

In the present invention, because a promoter can be arranged upstream of a sense strand cDNA, an RNA to be transcribed is a sense strand RNA (i.e. a sequence same as mRNA). Thus, a protein can be directly expressed by applying an appropriate expression system as long as the RNA transcribed in this manner contains a translation initiation point. An expression system in the present invention means a system capable of translating the above RNA into a protein. The system can be *in vitro* or *in vivo* system. In cDNAs based on the present invention, two main kinds of translation initiation points may be present. One is derived from an mRNA which was used as a template, and the other is a translation initiation point provided by an artificially added nucleotide sequence. If a translation initiation point is derived from an mRNA, the cDNA is highly possible to be full-length cDNA. In contrast, the case of a translation initiation point provided by the artificially added nucleotide sequence is described in detail below as an embodiment in which a cDNA of the present invention is expressed as a fusion protein. In either case, a protein can be translated based on an RNA transcribed from a cDNA in the case of containing a translation initiation point. If cDNAs are a library, proteins obtained based on the library construct a library. The present invention also provides a protein library obtained in this manner.

Proteins obtained based on a cDNA library become a protein library reflecting the condition of the cDNA library. As an expression system capable of translating RNAs transcribed *in vitro* into proteins, kits for expression using a rabbit reticulocyte lysate or a wheat embryo extract are available in the market and can be used. By using these cell-free expression systems, proteins can be obtained in a very small amount but in the soluble form and even in the condition similar to the natural structures. The obtained protein library can be used as a source for screening drug targets, or analytic materials for examining the cellular condition by changes of expressed proteins.

A library of sense strand RNA transcribed from a cDNA library of the present invention can be expressed directly in cells. A biological activity in a protein encoded by a sense strand RNA in cells can be expressed by introducing the sense strand RNA into the cells (Henle, K. J. et al., Expression of thermotolerance following microinjection of poly (A) RNA isolated from thermotolerant CHO cells., Int. J. Hyperthermia., 1990, 6 (6), 1041-1051). As a means for introducing an RNA into cells, a microinjection method is generally appropriate, but any methods capable of introducing a nucleic acid molecule into cells, for example, a lipofection and a particle gun, can be used. A protein encoded by an introduced gene is produced by a protein synthesis system in cells using a sense strand RNA introduced into cells as a template, and biological activity thereof is expressed.

For example, when an experimental system for examining effects of a physiological condition on cells using cells on the transient stage in development, limited diseased tissues, and so on as a material, an assay system for screening, for example drugs reacting in such a physiological condition, and such are attempted to construct, the amount of the materials is limited. Thus, preparation with high reproducibility and in a large amount is extremely difficult. In such a case, a cDNA library synthesized from cells or tissues in the corresponding physiological condition based on the present invention is useful. More specifically, by introducing an RNA library obtained using this library as a master library into an appropriate cell system, a model system virtually reproducing the status of target cells can be constructed. By using a cDNA library of the present invention, a necessary assay system can be provided easily and highly reproducibly.

In the following, an embodiment in which a gene encoding another protein is arranged as a nucleotide sequence to be artificially added is described. In the present invention, a gene encoding another protein can be arranged as the above known nucleotide sequence to be artificially added. Especially in an embodiment to arrange the above promoter sequence, when a gene encoding another protein is linked to upstream of a cDNA, a protein (unknown) encoded by the cDNA is expressed as a fusion protein with a combined protein. As a protein to be combined, for example, a protein comprising a specific binding activity, a protein providing a detectable signal, or a protein comprising a biological activity can be used.

As a protein comprising a specific binding activity, for example, a protein having a known binding activity such as protein A, histidine tag, or HA tag can be used. A protein library of the present invention fused with these proteins can be captured on a solid phase using corresponding ligands. The captured libraries can be used for screening ligands and receptors, or signal transduction systems.

As a protein providing a detectable signal, for example, a fluorescent protein, such as green fluorescent protein (GFP), or an enzyme protein, such as β galactosidase or peroxidase, can be used. A protein library of the present invention fused with these proteins also can be used for screening ligands and receptors, or signal transduction systems by performing a binding reaction with a candidate compound.

When a gene encoding these proteins is arranged between the above promoters and cDNA, a cDNA library can be also obtained by the same methods described above. A relatively long nucleotide sequence is occasionally added to mRNAs. If a sequence to be added is long, for example, a sequence added to an mRNA must be a long sequence encoding another protein. However, an immobilized probe capturing this may be only a part corresponding to a promoter. Alternatively, a method in which only the 3'-side of a long sequence to be added is added, and the rest part is supplemented at the synthesis of the second strand (sense strand) (Figure 6) can be adopted. In this figure, only a part of a sequence to be added is provided in the first strand. The rest part is supplemented as a part of an immobilized primer for synthesizing the second strand. At the synthesis of the second strand (a sense strand) in this embodiment, a reaction which extends toward the 3'-direction using the primers in which an antisense strand is immobilized as a template progresses, simultaneously with the progression of the synthetic reaction of the second strand (a sense strand).

A sequence of a gene encoding a protein requires an open reading frame which contains a codon capable of initiating a translation and which does not contain a stop codon to form a fusion protein with a gene linked to the 3'-terminal in the same frame. In addition, to be immobilized as a fusion protein gene capable of expressing, the 5'-terminal of the sense strand cDNA is preferably present in the region encoding the protein. Thus, an incomplete length RNA source is rather suitable as an mRNA source. If the 5'-terminal of a linked sense strand cDNA is present within the region encoding a protein, a fusion protein gene in frame is formed at the probability of one third.

By using this principle, an incomplete-length cDNA (not comprising a translation initiation point) can be collected in the form capable of expressing. More specifically, as a known nucleotide sequence to be artificially added, one containing at least a translation initiation point is prepared. An incomplete-length cDNA is linked thereto. In other wards, only a translation initiation point is artificially provided. An RNA which transcribed the cDNA obtained in this manner encodes am amino acid sequence in frame at the probability of one third same as the case of encoding the above fusion protein.

As a sense strand is immobilized in the cDNA library of the present invention, a cDNA subtraction method is possible using the sense strand as a driver. By hybridizing, as a tester, the first strand cDNA synthesized by a given method with a cDNA library of the present invention, cDNAs comprising sequences which are included only in the first strand cDNAs can not hybridize and remain in a liquid phase. By separating this from a solid phase, a subtraction can be easily conducted. By using a cDNA library of the present invention as a control, a subtraction for various subjects can be achieved repetitively, and highly reproducible studies can be conducted. More specifically, for example, a cDNA library derived from normal cells is solid-phased based on the present invention. Using this cDNA library, drug candidate compounds can be screened by subtracting cDNAs of cells treated with drug candidate compounds. Alternatively, by subtracting cDNAs of abnormal cells such as oncocytes, genes specific to abnormal cells can be screened. Moreover, by immobilizing a cDNA library derived from hepatocytes, and subtracting cDNAs derives from other organs, organ-specific genes can be selected. In any cases, a working efficiency which has never achieved for known subtraction methods can be achieved as a cDNA library of the present invention enables subtraction of the first strand cDNA due to the immobilized sense strand. In addition, in a preferable embodiment, a more reliable subtraction can be expected due to the high maintenance of diverse full-length cDNAs. Moreover, repeated uses are advantageously possible due to the immobilization.

Genes can be cloned using a cDNA library of the present invention as a source. For example, an mRNA library synthesized *in vitro* based on a cDNA library of the present invention can be screened by a standard gene cloning method. When a library for performing such a screening method is commercially provided, the following embodiments can be proposed.

An mRNA library synthesized based on a cDNA library of the present invention

A cDNA library synthesized from the mRNA

A DNA library synthesized based on a cDNA library of the present invention

Alternatively, when a structure of a gene to be obtained has been already known, a target gene can be obtained by directly amplifying by PCR from a cDNA library of the present invention, or by conducting RT-PCR using, as a template, mRNAs synthesized from the cDNA library of the present invention.

In any case, as repeatedly described, such various embodiments are possible because the primary library with diverse full-length cDNAs can be produced due to an efficient immobilization of full-length cDNAs in the cDNA library of the present invention. In known methods, an immobilized cDNA library with highly diverse full-length cDNAs is difficult to produce. Thus, the diversity of full-length cDNAs is extremely lowered after the synthesis of the secondary or tertiary library by using the cDNA library as a master library, and a quality required as genetic resources can not be maintained. In contrast, in the present invention, a library of cDNAs (or mRNAs) equivalent to full-length mRNAs derived from samples can be theoretically synthesized infinitely. In other words, a cDNA library of the present invention has characteristics desirable as a master library for cloning.

In the following, specific manipulations for constructing a cDNA library in which the 5'-side of the a sense strand is immobilized based on the present invention and for preparing the secondary library or an mRNA library using this cDNA library as a primary library are illustrated. In this example, as a method for adding an artificial sequence to the 5'-side of an mRNA, the oligo-CAP method is applied, but the present invention is not limited to this example. Basic manipulations of the oligo-CAP method follow the method described in Suzuki, J. and Sugano, S., "cDNA cloning," Yodosha, 1996, 46-51.

To 5 µg (84 µL) of poly (A)⁺ RNA extracted from a cellular sample, the following reagents are added and incubated at 37°C for 30 min. The reaction solution was treated with phenol-chloroform twice and RNAs are collected by ethanol precipitation.

| | |
|---|---|
| 10 x BAP buffer (Takara Shuzo) | 10 µL |
| Alkaline phosphatase (derived from bacteria, Takara Shuzo) | 3 µL |
| Ribonuclease inhibitor | 3 µL |

The collected precipitate is dissolved in 75 µL of distilled water and the following reagents are added thereto and incubated at 37°C for 30 min. The reaction solution is treated with phenol-chloroform and RNAs are collected by ethanol precipitation.

| | |
|---|---|
| 5 x TAP buffer | 20 µL |
| Tobacco acidic pyrophosphatase (Shinshi, H. et al., Biochem., 1976, 15, 2185) | 3 µL |
| Ribonuclease inhibitor (Takara Shuzo) | 2 µL |
| * 5 x TAP buffer: 250 mM sodium acetate (pH 5.5) 5 mM EDTA (pH 8.0) 50 mM 2-mercaptoethanol | |

The collected precipitate is dissolved in 6.4 µL of distilled water and the following reagents are added thereto and incubated at 16°C for 3 hours. The reaction solution is treated with phenol-chloroform and RNAs are collected by ethanol precipitation. An oligo RNA to be added here is an oligonucleotide comprising a nucleotide sequence to be artificially added. This nucleotide sequence is used as a synthetic oligo RNA, for example, comprising a SfiI cleavage sequence close to the 5'-terminal and a strand length capable of annealing with a complementary strand under stringent conditions as a whole.

| | |
|---|---|
| 10 x RNA Ligation Buffer (Takara Shuzo) | 10 µL |
| 25 mM MgCl₂ | 20 µL |
| 24 mM ATP | 2.1 µL |
| Oligo RNA (100 ng/µL) | 4 µL |
| 50% polyethylene glycol 8000 | 50 µL |
| T4 RNA ligase (Takara Shuzo) | 5 µL |
| Ribonuclease inhibitor (Takara Shuzo) | 2.5 µL |

The collected precipitate is dissolved in 50 µL of TE buffer, and non-reacted oligo-RNA is removed by a spun column (Pharmacia Size Sep 400). The obtained RNA fraction is collected by ethanol precipitation. By the above manipulations, an artificial sequence is added specifically to the 5'-side of a full length mRNA. Then, the first strand cDNA is synthesized using the mRNA in which this artificial sequence has been added as a template. The collected RNA is dissolved in 21 µl of distilled water, and the following reagents are added thereto and incubated at 16°C for 1 hour and then at 42 °C for 1 hour. The oligo dT adapter to be used at this time comprises a SfiI recognition sequence at the 5'-terminal.

| | |
|---|---|
| 5 x First Strand Buffer (Gibco BRL) | 10 µL |
| 0.1 M DTT | 6 µL |
| Mixture of 5 mM dATP, dTTP, dCTP, and dGTP | 8 µL |
| Oligo dT adapter (5 pmol/µL) | 2 µL |
| Superscript II (Gibco BRL) | 2 µL |
| Ribonuclease inhibitor (Takara Shuzo) | 1 µL |

To the reaction solution, 50 µL of distilled water is further added. The solution is treated with phenol-chloroform, and 2 µL of 0.5 M EDTA and 15 µL of 0.1 N NaOH are added thereto and further incubated at 65°C for 1 hour. After the reaction, 20 µL of 1 M Tris-HCl (pH 7.8) is added to the reaction solution and the first strand cDNA is purified by a spun column (Pharmacia Size Sep 400). cDNAs are collected by the ethanol precipitation and dissolved in 80 µL of distilled water. The first strand cDNAs collected here have the nucleotide sequence complementary to a nucleotide sequence artificially added to mRNA at the 3-side. Using the first strand cDNA as a template, an immobilized cDNA library of the present invention is synthesized.

The first strand cDNA (80 µL) is transferred to a tube (GenePlates, AGCT Inc.) in which an oligo DNA complementary to a sequence added to the 3'-terminal of the first strand cDNA has been immobilized. The first strand cDNA is incubated at 65°C for 10 min, and then at 12°C for 30 min for annealing with the immobilized oligo DNAs and the following reagents are added thereto and incubated at 30°C for 30 min.

| | |
|---|---|
| 5 x T4 polymerase Buffer (Takara Shuzo) | 10 µL |
| 5 mM dATP, dTTP, dCTP, dGTP mixture | 8 µL |
| T4 DNA polymerase (Takara Shuzo) | 2 µL |

After the reaction, the supernatant is removed, the tube is washed with distilled water, and 50 µL of TE buffer is added thereto. On the inner wall of the tube, the second strand cDNA (sense strand cDNA) is bound with its 5'-side immobilized to construct an immobilized cDNA library of the present invention. Then the synthetic manipulation of the secondary cDNA library in which this cDNA library is a primary library is described.

To an immobilized cDNA library of the present invention, the following regents are added and DNAs are amplified at 95°C for 5 min; "at 95°C for 1 min, at 58°C for 1 min, and 72°C for 10 min" for 15 cycles; and at 72°C for 10 min, and cooled to 4°C. As a 5'-primer, a sequence same as an oligo-DNA immobilized in the tube, and as a 3'-primer, an oligo dT primer may be used.

| | |
|---|---|
| Distilled water | 52.4 µL |
| 3.3 x PCR Buffer (Perkin-Elmer) | 30 µL |
| 2.5 mM dNTP mixture solution | 8 µL |
| 2.5 mM magnesium acetate | 4.4 µL |
| 5'-primer (10 pmol/µL) | 1.6 µL |
| 3'-primer (10 pmol/µL) | 1.6 µL |
| GeneAmp DNA Polymerase (Perkin-Elmer) | 2 µL |

The supernatant is transferred to a new tube, treated with phenol-chloroform, and precipitated with ethanol, and the precipitate is dissolved in 89 µL of distilled water. The following reagents are added thereto and incubated at 50°C for 3.5 hours.

| | |
|---|---|
| Buffer #2 (New England Biolabs) | 10 µL |
| Bovine serum albumin (x 100) (New England Biolabs) | 1 µL |
| SfiI (New England Biolabs) | 2 µL |

The reaction solution is treated with phenol-chloroform and precipitated with ethanol, and the precipitate is dissolved in 50 µL of TE buffer. The agarose gel electrophoresis is conducted and shorter fragments are removed by excising them from the gel, and the collected DNA is dissolved in 20 µL of distilled water. This DNA is ligated with a cohesive end of DraIII-digested pME18SFL3 vector (GenBank Acc. No. AB009864) fragment, which is complementary to a cohesive end remaining in the above DNA fragment digested with SfiI, with T4 DNA ligase, and *E. coli* is transformed with the vector obtained. In this manner, the secondary vector library in which a cDNA library of the present invention is the primary library can be constructed. Alternatively, in the case of using a promoter sequence of an RNA polymerase as the above sequence to be artificially added, a sense strand RNA library can be synthesized by the *in vitro* transcription reaction. Following is a description for synthesizing an RNA library.

To the immobilized primary library of the present invention, the following reagents are added and incubated at 37°C for 10 min. After the reaction, the supernatant is transferred to a new tube, treated with phenol-chloroform and precipitated with ethanol. A precipitate is dissolved in 50 µL of distilled water. Thus, an RNA library transcribed using a cDNA of the present invention as a template is collected.

| | |
|---|---|
| Distilled water | 76.8 µL |
| 10 x T7 Pol. Buffer (Takara Shuzo) | 10 µL |
| 5 mM rATP, rUTP, rCTP, rGTP mixture solution | 8 µL |
| 2.5 mM magnesium acetate | 4.4 µL |
| T7 RNA polymerase (Takara Shuzo) | 2 µL |

### Brief Description of the Drawings

Figure 1 schematically shows a principle for synthesizing a cDNA immobilized at the 5'-side of the sense strand by the present invention.

Figure 2 schematically shows a principle for synthesizing a cDNA immobilized at the 5'-side of the sense strand by the present invention, indicating an embodiment in which a known nucleotide sequence is artificially added to the 3'-terminal of the first strand cDNA.

Figure 3 schematically shows of a principle for synthesizing a cDNA immobilized at the 5'-side of the sense strand by the present invention, indicating an embodiment in which a complementary sequence of a known nucleotide sequence is artificially added to the 5'-terminal of an mRNA.

Figure 4 schematically shows a principle for synthesizing a cDNA immobilized at the 5'-side of the sense strand by the present invention, indicating an embodiment in which a complementary sequence of a known nucleotide sequence is artificially added to the CAP structure at the 5'-terminal of an mRNA. A full-length cDNA can be specifically immobilized by using a reaction specific to the CAP structure at the 5'-terminal.

Figure 5 schematically shows of a principle for synthesizing a cDNA immobilized at the 5'-side of the sense strand by the present invention, showing that the arrangement to upstream of the sense strand is possible by using a promoter as a known sequence.

Figure 6 schematically shows a principle for synthesizing a cDNA immobilized at the 5'-side of the sense strand by the present invention, showing a variation in the case of adding a long nucleotide sequence.

Figure 7 shows an electrophoretic photograph of a PCR amplification fragment of a full length EF1α gene.

Figure 8 shows an electrophoretic photograph which confirms that the secondary cDNA library can be obtained by being repeatedly replicated and collected by using the DNA polymerase Klenow fragment from a gene plate in which the 5'-terminal of the sense strand of a full-length cDNA is immobilized.

Figure 9 shows a photograph showing the result in which a sense strand RNA synthesized *in vitro* using a full-length cDNA fragment of EF1α immobilized at the 5'-terminal side of the sense strand as a template was detected by Northern blot.

### Best Mode for Carrying out the Invention

### Example 1: Immobilization of the 5'-terminal of a sense strand in a specific full-length cDNA

As an example of immobilizing the 5'-terminal of a sense strand in a specific gene, a sense strand of human Elongation Factor-1α (EF1α, GenBank Acc. No. E02628) full-length cDNA clone was immobilized. As a gene fragment to be immobilized, an EF1α full-length cDNA fragment amplified by PCR using, as a template, the first strand cDNA library obtained by the oligo CAP method, described in Suzuki, J. and Sugano, S., "cDNA cloning," Yodosha, 1996, 46-51, was used. For PCR primers, the oligo CAP linker primer FL3-666 added to the 5'-terminal of EF1α gene (sequence 5'-AGC ATC GAG TCG GCC TTG TTG -3'; SEQ ID NO: 4) and EF1α primer EF1-8R designed based on a sequence close to the 3'-terminal of EF1α cDNA (about 1.7 kb) (sequence 5'-TGG GTC TCA AAA TTC TGT GAC-3'; SEQ ID NO: 5) were used. To 0.5 µl of a template DNA solution (about 10 ng), 5.0 µl of 10 x PCR buffer [100 mM Tris-HCl (pH 8.3), 500 mM potassium chloride, 150 mM magnesium chloride], 4.0 µl of each 25 mM dNTP, and 1 µl each of 5 pmol/µl of primers were added and sterile water was further added thereto to 49 µl. After 1.0 µl (5.0 U) of Takara Taq DNA polymerase (Takara Shuzo) was added thereto, PCR reaction was conducted by incubating at 95°C for 5 min; and at 95 °C for 1 min, at 60°C for 1 min, and at 72°C for 2 min for 30 cycles using Perkin-Elmer Model 9600 Thermal Cycler. This PCR amplified a 1750 bp fragment containing almost full-length of EF1α gene. After PCR, the solution was treated with phenol-chloroform, precipitated with ethanol, and dissolved in 20 µl of TE buffer. This whole amount was electrophoresed on a 1.0% (w/w) agarose gel (1 x TAE buffer) and the amplified DNA fragment was collected by using Gene Clean (Bio 101). The collected fragment was dissolved in 20 µl of TE buffer.

To the collected EF1α full-length cDNA fragment solution, proteinase K (Boehringer Mannheim, the final concentration of 100 µg/ml) and sodium lauryl sulfate (SDS) (the final concentration of 0.5%) were added and treated at 37°C for 60 min. This solution was further treated with phenol-chloroform, and precipitated with ethanol, and the collected DNA was dissolved in 20 µl of TE buffer. The DNA obtained in this manner was loaded to a well of Gene Plate [AGCT, Inc. (Irvine, CA, USA)] on which T7-oligo CAP linker primer oligo DNA (sequence 5'-GTAATACGAC TCACTATAGG GAGCATCGAG TCGGCCTTGT TGGCCTACTG G-3'; SEQ ID NO: 6) was immobilized mediated by a spacer, and the 5'-terminal of a sense strand was immobilized by PCR. PCR was conducted by using GeneAmp XL PCR Kit (Perkin-Elmer) in a system whose whole amount was 50 µl. PCR was conducted by adding 1.0 µl each of 5 pmol/µl FL3-666 primer (SEQ ID NO: 4) and EF1-8R primer (SEQ ID NO: 5), 4.0 µl of dNTP solution (2.5 mM each), 15 µl of 3.3 x XL PCR buffer (Perkin-Elmer), 2.2 µl of 25 mM (CH₃COO)₂Mg, and water to 1 µl of DNA fragment solution containing EF1α full-length cDNA (in which about 2 ng of DNA fragment is contained) to 49 µl, and then, 1.0 µl (2 U)of rTth DNA polymerase (Perkin-Elmer) was added thereto. The reaction was conducted by Perkin-Elmer Model 9600 Thermal Cycler by incubating at 95°C for 5 min, and then at 95°C for 1 min, at 60°C for 1 min, and at 72°C for 2 min for 25 cycles. After PCR, the supernatant was collected and transferred to another container and stored. On the other hand, a well of the Gene Plate on which EF1α full-length cDNA fragment was immobilized in this manner was washed twice with 120 µl of the plate wash buffer [0.5 M sodium chloride, 10 mM Tris-HCl (pH 8.0), 1 mM EDTA].

### Example 2: Obtaining secondary amplified fragments from a plate on which the 5'-terminal of a sense strand in EF1α full-length cDNA is immobilized

To a well of the Gene Plate on which EF1α full-length gene fragments prepared in Example 1 was immobilized, 39 µl of the Klenow reaction buffer [as the final concentrations, 0.2 mM each of dNTP, 10 mM Tris-HCl (pH 7.5), 7 mM magnesium chloride, 0.1 mM dithiothreitol] containing 4 pmol each of T7 primers (sequence 5'-GTAATACGACTCACTATAGGG-3'; SEQ ID NO: 7) and EF1-8R primer (SEQ ID NO: 5) were added, and incubated at 94°C for 30 sec. The temperature of the reaction solution in the well was cooled to 30°C, and 1 µl (4U) of DNA polymerase Klenow fragment (Takara Shuzo) was added thereto and reacted at 30°C for 3 hours. After the reaction, the supernatant was collected and stored after transferred to another container. The well of the Gene Plate on which cDNA was immobilized was washed with 120 µl of the plate wash buffer (0.5 M sodium chloride, 10 mM Tris-HCl (pH 8.0), 1 mM EDTA) twice by gently pipetting.

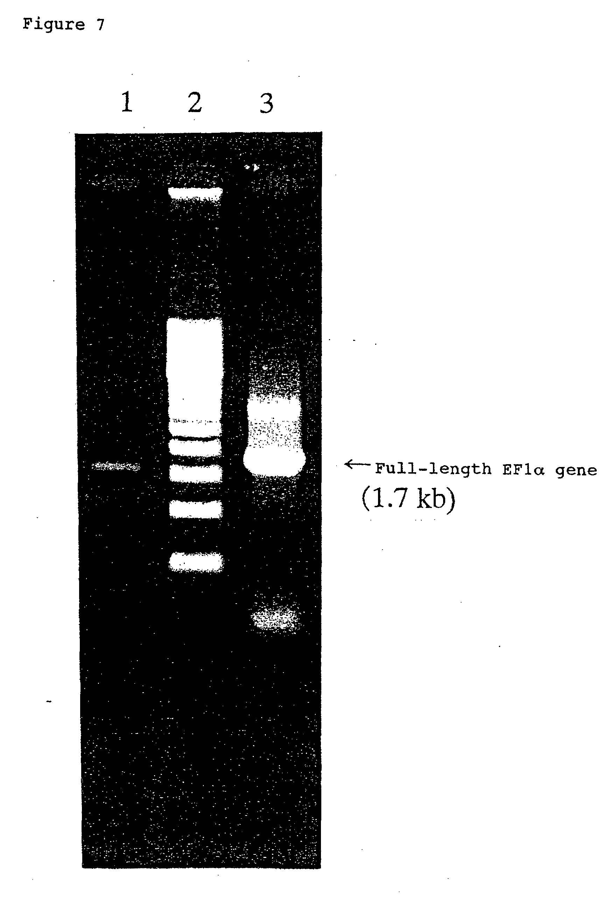

The Klenow reaction solution collected from the well of the Gene Plate on which EF1α full-length cDNA fragment was immobilized was treated with phenol-chloroform, precipitated with ethanol, and dissolved in 50 µl of TE buffer. T7 primer (SEQ ID NO: 7, 5 pmol/µl, 0.5 µl), 0.5 µl of 5 pmol/µl EF1-8R primer (SEQ ID NO: 5), 2.0 µl of 2.5 mM each of dNTP solution, 7.5 µl of 3.3 x XL PCR buffer (Perkin-Elmer), 1.1 µl of 25 mM (CH₃COO)₂Mg, and sterile water were added to 10 µl of the collected Klenow reaction solution to 24.5 µl, and 0.5 µl (1 U)of rTth DNA polymerase (Perkin Elmer) were added to conduct PCR. The reaction was conducted by Perkin-Elmer Model 9600 Thermal Cycler by incubating at 95°C for 5 min, and then at 95°C for 1 min, at 60°C for 1 min, and at 72°C for 2 min for 30 cycles. After the PCR, 10 µl of the product was electrophoresed on a 1.0% (w/w) agarose gel (1 x TAE buffer) and the amplification of the fragment was confirmed (Figure 7). By this electrophoresis, 1.7 kb EF1α full-length cDNA fragment replicated and collected by the reaction of DNA polymerase Klenow fragment using EF1α full-length cDNA immobilized in the well of the Gene Plate as a template was confirmed. The electrophoresed samples in each lane are shown below. The location of the band for the DNA fragment containing EF1α full-length cDNA (1.7 kb) was indicated at the right side by an arrow.
Lane 1: The DNA fragment containing EF1α full-length cDNA immobilized on the Gene Plate.
Lane 2: Size marker
Lane 3: PCR products obtained by amplifying the secondary amplified fragment replicated and collected using the EF1α full-length cDNA immobilized on the Gene Plate as a template with DNA polymerase Klenow fragment

This electrophoresis confirmed the 1.7 kb full-length cDNA fragment of EF1α replicated and collected by the reaction of DNA polymerase Klenow fragment using EF1α full-length cDNA immobilized in a well of the Gene Plate as a template.

### Example 3: Immobilization at the 5'-terminal side in the sense strand of a full-length cDNA library

From about 50 µg of poly (A)⁺ RNA obtained from NT2 cells by the method described in Sambrook, Molecular Cloning, Second Edition, 7.12 and 7.26, the first strand cDNA was prepared by the oligo CAP method described in Suzuki, J. and Sugano, S., "cDNA cloning," Yodosha, 1996, 46-51. An mRNA annealing to the first strand cDNA was removed by treatment with alkali, and the resultant was neutralized and dissolved in 40 µl of TE buffer for use. This first strand cDNA was immobilized on a solid phase using the DNA polymerase Klenow fragment.

After the solution containing the first strand cDNA was denatured by incubating at 65°C for 5 min, the total volume of the solution was adjusted to 50 µl, and the composition of the solution was adjusted to 0.5 M sodium chloride, 10 mM Tris-HCl (pH 8.0), and 1 mM EDTA as final concentrations. This solution was loaded to a well of the Gene Plate (AGCT, Inc.) on which T7-oligo CAP linker-primer oligo DNA (SEQ ID NO: 6) was immobilized through a spacer, and incubated at 16°C for 15 hours. A cDNA part complementary to the oligo CAP linker linked to the 5'-terminal of the first strand cDNA derived from full-length mRNA was sufficiently annealed with an oligo DNA comprising the same sequence as the oligo CAP linker immobilized in Gene Plate. Then, the supernatant was removed and the well was washed with 120 µl of the plate wash buffer [0.5 M sodium chloride, 10 mM Tris-HCl (pH 8.0), 1 mM EDTA] twice. To this well, 39 µl of Klenow reaction buffer (as final concentrations, 0.2 mM each of dNTP, 10 mM Tris-HCl (pH 7.5), 7 mM magnesium chloride, 0.1 mM dithiothreitol) and 1 µl (4 U) of the DNA polymerase Klenow fragment (Takara Shuzo) were added, and reacted at 30°C for 3 hours. Using the first strand cDNA annealed to the oligo DNA immobilized as a template, and the oligo DNA in which the 5-terminal was immobilized as a primer, the second strand cDNA was thus synthesized. At this time, the first strand cDNA complementary to the T7 promoter sequence upstream of the immobilized oligo CAP linker was synthesized simultaneously using the annealed first strand cDNA as a primer (refer to Figure 6). In this manner, a library of full-length double strand cDNA in which the 5'-terminal of the sense strand was immobilized on the Gene Plate was obtained. After the synthetic reaction of the second strand cDNA, the supernatant was removed, and transferred to another container for storage. After the supernatant was removed, the well on which the double strand cDNA was immobilized was washed with the wash buffer (0.5 M sodium chloride, 10 mM Tris-HCl (pH 8.0), 1 mM EDTA) twice.

### Example 4: Repetitive replication and isolation. of the secondary library of double strand full-length cDNA using the primary library of a full-length cDNA immobilized in the 5'-terminal side of the sense strand as a template

Using the primary library of full-length cDNA immobilized in the 5'-terminal of the sense strand as a template, it was confirmed that the secondary library of double strand full-length cDNA could be synthesized. As the primary library of full-length cDNA, the library of cDNA immobilized at the 5'-side of the sense strand on the wells of the Gene Plate in Example 3 was used. Using a T7 sequence linked to 5'-terminal of the sense strand side of cDNA and a sequence of poly A part present in the 3'-terminal side as primers, DNA was replicated using the DNA polymerase Klenow fragment, and the secondary library of double strand full-length cDNA was isolated.

To a well of the Gene Plate, 0.2 pmol each of T7 primer (SEQ ID NO: 7) and FL3-705 primer (sequence 5'-GCG GCT GAA GAC GGC CTA TGT-3'; SEQ ID NO: 8) and the Klenow reaction buffer [as final concentrations 0.2 mM each of dNTP, 10 mM Tris-HC1 (pH 7.5), 7 mM magnesium chloride, 0.1 mM dithiothreitol] were added, adjusted to the volume of 39 µl, and incubated at 94 °C for 30 sec. The temperature of the reaction solution in the well of the Gene Plate was cooled to 30°C, and 1 µl (4U) of the Klenow fragment (Takara Shuzo) was added and reacted at 30°C for 3 hours. After the reaction, the supernatant was collected, and the secondary library of double strand full-length cDNA replicated using an immobilized cDNA as a template was obtained. The used well was washed with the wash buffer [0.5 M sodium chloride, 10 mM Tris-HCl (pH 8.0), 1 mM EDTA] twice. By repeating this manipulation, the secondary library of double strand full-length cDNA replicated by using a primary library of immobilized full-length cDNA as a template, was synthesized. The Klenow reaction solution was collected after each reaction, treated with phenol-chloroform, precipitated with ethanol, and dissolved in 40 µl of TE buffer.

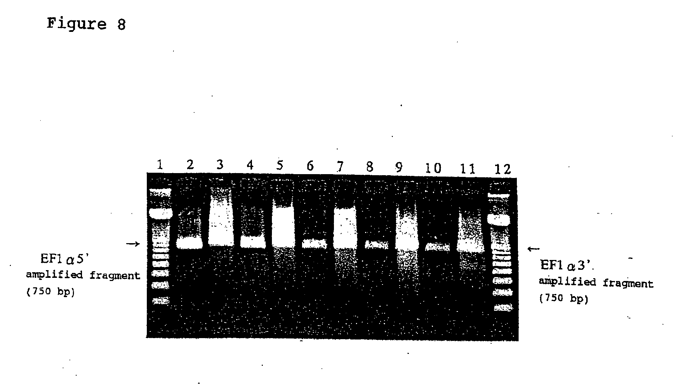

Using the secondary library of double strand full-length cDNA obtained in this manner as a template, with the combination of T7 primer (SEQ ID NO: 7) and EF1α primer EF1-1R (SEQ 5'-TGC TAC TGT GTC GGG GTT GTA-3'; SEQ ID NO: 9), or the combination of EF1α primer EF1-3F (sequence 5'-CCT GAA CCA TCC AGG CCA AAT-3'; SEQ ID NO: 10) and FL3-705 primer (SEQ ID NO: 8), PCR was conducted. If the secondary library of double strand full-length cDNA is collected, the 5'-terminal fragment of EF1α gene, 750 bp, and the 3'-terminal fragment of EF1α gene, 750 bp, should be amplified by this PCR. PCR was conducted by adding 2.5 µl of 10 x PCR buffer (100 mM Tris-HCl (pH 8.3), 500 mM potassium chloride, 150 mM magnesium chloride), 2.0 µl of 25 mM dNTP, 0.5 µl each of 0.5 pmol/µl primers, and sterile water to 6.0 µl of the collected secondary library of double strand full-length cDNA to 24.5 µl, and further adding 0.5 µl (2.5 U) of Takara Taq DNA polymerase (Takara Shuzo) thereto. The reaction was conducted by using Perkin-Elmer Model 9600 Thermal Cycler, by incubating at 95°C for 5 min, and then at 95°C for 1 min, at 58°C for 1 min, and at 72°C for 1 min for 35 cycles. After PCR, a part of the product was electrophoresed on a 2.0% (w/w) agarose gel (TBE buffer) to examine amplification of each fragment (Figure 8). As a result, even when the secondary full-length cDNA library was repeatedly replicated until five times, each of the 5'-terminal and the 3'-terminal fragments of EF1α gene was confirmed to be amplified.

Using the secondary cDNA replicated and collected repeatedly from the Gene Plate as a template to amplify the 5'-terminal fragment (750 bp) and the 3'-terminal fragment (750 bp) of EF1α gene, the secondary library of full-length cDNA was shown to be obtained in the supernatant. The locations of bands of the 5'-terminal fragment (750 bp) and the 3'-terminal fragment (750 bp) were shown with the arrows at the left and right sides, respectively. Samples on each lane were described below.
Lane 1: Size marker
Lanes 2, 4, 6, 8, and 10: The result of PCR for amplifying the 5'-terminal fragment (750 bp) of EF1α gene replicated and collected using as a template the secondary cDNA library replicated and collected by the repetitive DNA polymerase Klenow fragment reaction from the Gene Plate on which the primary full-length cDNA was immobilized
Lanes 3, 5, 7, 9,and 11: The result of PCR for amplifying the 3'-terminal fragment (750 bp) of EF1α gene replicated and collected using as a template the secondary cDNA library replicated and collected by the repetitive DNA polymerase Klenow fragment reaction from the Gene Plate on which the primary full-length cDNA was immobilized
Lanes 2 and 3: The result of PCR using the collected solution of the first replication conducted on the Gene Plate as a template
Lanes 4 and 5: The result of PCR using the collected solution of the second replication conducted on the Gene Plate as a template
Lanes 6 and 7: The result of PCR using the collected solution of the third replication conducted on the Gene Plate as a template
Lanes 8 and 9: The result of PCR using the collected solution of the forth replication conducted on the Gene Plate as a template
Lanes 10 and 11: The result of PCR using the collected solution of the fifth replication conducted on the Gene Plate as a template
Lane 12: Size Marker

These findings confirmed that the 5'-terminal of the sense strand of EF1α full-length cDNA was immobilized on the Gene Plate. From these results, on the wells of the primary full-length cDNA library immobilized at the 5'-terminal of the sense strand, the secondary double strand full-length cDNA library was confirmed to be obtained by performing the DNA replication reaction using the DNA polymerase Klenow fragment with the T7 sequence linked to the 5'-terminal of the sense strand cDNA and the sequence of poly A present at the 3'-terminal side as primers.

### Example 5: RNA synthesis in vitro using the primary library of full-length cDNA immobilized at the 5'-terminal side of the sense strand as a template

By the methods of Examples 1 and 2, a DNA fragment containing full-length EF1α gene was immobilized on a well of the Gene Plate (AGCT, Inc.) on which the T7-oligo CAP linker primer (SEQ ID NO: 6) was immobilized through a spacer. In the well of the Gene Plate obtained in this manner, using AmpliScribe T7, T3, SP6 High Yield Transcription Kit (Epicentre), RNA synthesis was conducted *in vitro.* An RNA was synthesized *in vitro* by following the manual of the kit, by adding, so that the total volume became 50 µl, the kit-attached reaction buffer, 7.5 mM ATP, 7.5 mM GTP, 7.5 mM CTP, 7.5 mM UTP, 10 mM dithiothreitol (final concentrations), and 5 µl AmpliScribe T7 enzyme solution to the wells on which full-length EF1α gene used as a template was immobilized and by reacting at 37°C for 2 hours. After the RNA synthesis reaction, the supernatant was collected from the well, treated with phenol-chloroform, and precipitated with ethanol. A part of the reaction solution obtained in this manner was subjected to the Northern hybridization experiment of Example 7.

### Example 6: Preparation of a probe for detecting an RNA synthesized on the immobilized plate

By following the method in Japanese Patent Application No. Hei 10-324201 (filed on November 13, 1998), the synthetic oligo DNA containing an inner sequence of EF1α gene (EF1-7R) (sequence 5'-TGG TCC ACA AAA CAT TCT CCT-3'; SEQ ID NO: 11) was labeled with digoxigenin (DIG, Boehringer Mannheim). The synthetic oligo DNA (100 pmol) was dissolved in the whole amount of 19 µl of the tailing buffer [as final concentrations, 200 mM sodium cacodylate, 25 mM Tris-HCl (pH 6.6), 0.25 mg/ml bovine serum albumin solution, 5 mM cobalt chloride solution, 50 µM DIG-dUTP solution, 0.5 mM dITP]. One microliter (2.5 U) of terminal transferase (Boehringer Mannheim) was added thereto, and the mixture was reacted at 37°C for 15 min. The mixture was transferred on ice after the reaction, and EDTA (pH 8.0) at a final concentration of 40 mM was added thereto to terminate the reaction. The labeled oligo DNA was precipitated by adding 0.01 µl of 20 mg/ml glycogen, 2.5 µl of 4 M lithium chloride, and 75 µl of ethanol, and dissolved in 100 µl of sterile water.

### Example 7: Detection of EF1α gene RNA synthesized in vitro on the immobilized Gene Plate

A sense strand RNA of EF1α gene to be used as a positive control was prepared by the standard *in vitro* RNA synthesis. The DNA fragment containing full-length EF1α gene prepared in Example 1 was cloned using TOPO TA Cloning Kit (Invitrogen). Among the obtained recombinant plasmids, plasmids in which EF1α was linked downstream of the T7 promoter in the sense direction were selected. By using this plasmid as a template, the EF1α full-length cDNA fragment containing the T7 promoter upstream was obtained by PCR. As PCR primers, T7 primer (SEQ ID NO: 7) and EF1α primer EF1-8R (SEQ ID NO: 5) were used. To 0.5 µl of the template DNA solution (about 10 ng), 5.0 µl of 10 x PCR buffer (100 mM Tris-HCl (pH 8.3), 500 mM potassium chloride, 150 mM magnesium chloride), 4.0 µl of each 25 mM dNTP, and 1.0 µl each of 5 pmol/µl primers were added, and further sterile water was added thereto to 49 µl. Then, 1.0 µl (5.0 U) of Takara Taq DNA polymerase (Takara Shuzo) was added thereto to conduct PCR. PCR was conducted by Perkin-Elmer Model 9600 Thermal Cycler by incubating at 95°C for 5 min, and reacting for 30 cycles at 95°C for 1 min, at 60°C for 1 min, and at 72°C for 2 min. By this PCR, a 1750 bp fragment comprising T7 promoter upstream and containing almost full-length of EF1α gene was amplified. After PCR, the solution was treated with phenol-chloroform, precipitated with ethanol, and dissolved in 20 µl of TE buffer. A whole amount of this solution was electrophoresed on a 1.0% (w/w) agarose gel (1 x TAE buffer), and the amplified DNA fragment was collected by Gene Clean (Bio 101). The collected fragment was dissolved in 20 µl of TE buffer.

To the collected EF1α full-length cDNA fragment solution, proteinase K (Boehringer Mannheim, the final concentration of 100 µg/ml) and sodium lauryl sulfate (SDS) (the final concentration of 0.5%) were added and treated at 37°C for 60 min. This solution was further treated with phenol-chloroform and precipitated with ethanol, and the collected DNA was dissolved in 20 µl of TE buffer. An RNA was synthesized *in vitro* using AmpliScribe T7, T3, SP6 High Yield Transcription Kit (Epicentre). The RNA synthetic reaction *in vitro* was conducted using the kit-attached reaction buffer, 7.5 mM ATP, 7.5 mM GTP, 7.5 mM CTP, 7.5 mM UTP, 10 mM dithiothreitol (final concentrations), and 5 µl of AmpliScribe T7 enzyme solution, at 37°C for 2 hours. After the RNA synthetic reaction, treatment with phenol-chloroform and ethanol precipitation were conducted and a part of the product was used as a positive control for the Northern hybridization experiment.

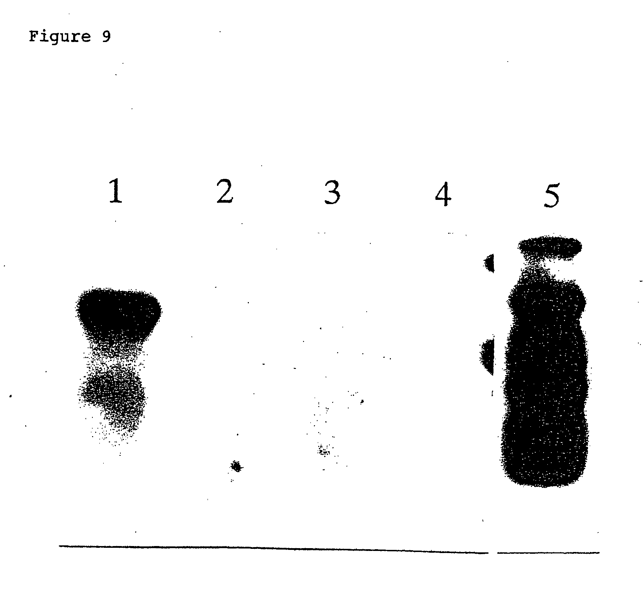

A part of RNA synthesized *in vitro* was electrophoresed on the 18% (v/v) formaldehyde-denatured 1.0% (w/w) agarose gel using the MOPS buffer (as final concentrations, 20 mM MOPS, 8 mM sodium acetate, 1 mM EDTA). The product was blotted onto a nylon membrane (Boehringer Mannheim) by following the capillary transfer method described in Sambrook, Molecular Cloning, Second edition, Cold Spring Harbor Press, 7.46, using 20 x SSC buffer, and then an RNA was immobilized using a Stratalinker UV Crosslinker (Stratagene). The filter prepared in this manner was prehybridized in the prehybridization buffer (0.9 x SSC, 1% blocking reagent, 0.1% sodium N-lauroyl sarcosinate, 0.02% SDS) at 68°C for 3 hours. Further hybridization was conducted in the hybridization buffer in which the oligo DNA probe of EF1α gene tail-labeled with DIG in Example 6 was added at a concentration of 10 pmol/ml to the prehybridization buffer at 52°C for about 20 hours. Then the membrane was washed with about 100 ml of the hybridization wash buffer (0.9 x SSC, 0.1% SDS) at 52°C for 15 min twice. By using the nylon membrane obtained in this manner and DIG Luminescence Detection kit (Boehringer Mannheim), the sense strand RNA of EF1α gene synthesized on the immobilized wells by following the manual supplemented with the kit was detected (Figure 9).

On the Gene Plate on which 5'-terminal of the sense strand of EF1α full-length cDNA was immobilized, an RNA was synthesized *in vitro.* As a control, the sample obtained by the similar experiment using about 50 ng of DNA fragment containing the T7 promoter upstream of EF1α full-length cDNA prepared by PCR as a template was loaded. In any cases, after treatment with DNase, electrophoresis was conducted. To confirm that the detected band was RNA, the samples treated with RNase at a final concentration of 0.2 µg/ml at 37°C for 10 min were loaded. Samples on each lane are as follows.
Lane 1: The supernatant of the product of *in vitro* RNA synthesis on the Gene Plate in which the 5'-terminal of the sense strand of EF1α full-length cDNA was immobilized, treated with DNase
Lane 2: The same as the sample of Lane 1, treated with RNase
Lane 3: The supernatant of the product of *in vitro* RNA synthesis using the DNA fragment comprising the T7 promoter upstream of the EF1α full-length cDNA prepared by PCR as a template, treated with DNase
Lane 4: The same sample as that of Lane 3, treated with RNase
Lane 5: Size marker

As a result, in the samples obtained by the RNA synthesis conducted on the Gene Plate on which the 5'-terminal of the sense strand of EF1α full-length cDNA was immobilized, the bands with the same length as the EF1α sense strand RNA synthesized by the standard *in vitro* reaction was detected. This signal disappeared by the RNase treatment, confirming that this band is RNA.

### Industrial Applicability

The present invention provides a cDNA of a novel structure in which the 5'-side of the sense strand cDNA was immobilized. By this characteristic, an immobilized cDNA library with the high immobilization efficiency of full-length cDNA and diverse full-length cDNA can be provided. The known immobilized cDNA libraries contain numerous incomplete cDNA due to the immobilization only by immobilizing a primer for synthesizing an antisense strand (the first strand). In the present invention, by artificially adding an known nucleotide sequence to the 3'-side of the first strand cDNA (an antisense strand), as a result, full-length cDNA can be immobilized at a high efficiency and an immobilized cDNA library with diverse full-length cDNA can be synthesized.

As a known artificially added nucleotide sequence in the present invention, a promoter capable of synthesizing an RNA *in vitro,* or a gene encoding a functional protein can be used. Moreover, these artificially added known nucleotide sequences are arranged upstream of the sense strand (the second strand), and thus, provide various uses. For example, in the case of using a promoter sequence, an mRNA library whose template is an immobilized cDNA library of the present invention can be prepared. In addition, because a cDNA library of the present invention is immobilized, RNAs with a same level of quality can be infinitely produced in theory by collection and reuse. By introducing the produced sense strand RNA containing a translation initiation codon into a living organism, a biological activity of the gene can be expressed in cells. For example, an expression RNA library produced from an immobilized expression cDNA library produced from a living organism in a specific condition can be a useful material for studying and analyzing responses of the living organism against the gene group expressed in the condition.

As the RNA obtained here is a sense strand in the present invention, a protein can be synthesized *in vitro.* Because a cDNA library containing a translation initiation codon can be immobilized, a protein library can be produced by the reaction for synthesizing proteins *in vitro.* The produced protein library can be a useful research material for, for example, proteome analysis and as a search source for various biologically active proteins, for example, medical products. An *in vitro* translation system provides proteins only in a very small amount but expresses the proteins in the condition similar to the natural condition due to extreme similarity to the protein synthesis system *in vivo.* While a size and a kind of protein molecules which can be expressed by, for example, phage library, are limited, an *in vitro* expression system does not have such limitations, and is suitable for the expression of diverse genes, such as a library. In addition, by inserting a gene encoding a specific protein between a promoter sequence and cDNA, a gene library for fusion proteins with a specific protein can be produced by using an immobilized cDNA library as a template.

A cDNA library provided by the present invention enables the synthesis of the secondary cDNA library using the library as a primary library. By using a cDNA library of the present invention as a primary library, theoretically all cDNAs are synthesized by conducting PCR using an oligo dT and an artificially added known nucleotide sequence as primers. A complex of cDNAs synthesized in this manner provides the secondary library of excellent quality, reflecting a population of full-length cDNA in the primary library. A library of the present invention can be theoretically reused over and over again due to the immobilization. In other words, a homogeneous library containing numerous full-length cDNAs can be continuously provided. In the cDNA libraries synthesized based on the known methods, the library is known to be difficult to be amplified while maintaining a population in mRNA, due to a very small amount of full-length cDNAs. Therefore, a cDNA library of the present invention is extremely useful for progressing researches in cDNA.

As described above, an industrial usefulness of the present invention is extremely high as a method for efficiently producing a sense strand RNA library, as a means for constructing a library of proteins encoded by the cDNA thereof, and a means for producing a full-length cDNA library with a stable quality.

## Claims

1. A cDNA library in which sense strand cDNAs are immobilized at the 5'-side.

2. The cDNA library of claim 1, wherein a common nucleotide sequence to cDNAs constituting the library is present at the 5'-terminal of sense strand cDNAs.

3. The cDNA library of claim 2, wherein the common nucleotide sequence is the sense sequence of a promoter specifically recognized by an RNA polymerase.

4. The cDNA library of claim 2, wherein the common nucleotide sequence encodes an arbitrary amino acid sequence and wherein the nucleotide sequence constitutes the same reading frame as the cDNAs.

5. The cDNA library of claim 1, wherein the sense strand cDNAs comprise a translation initiation codon.

6. The cDNA library of claim 5, wherein the translation initiation codon is derived from an mRNA.

7. A method for synthesizing a cDNA, wherein a known nucleotide sequence is artificially added to the 3'-terminal of a first strand cDNA and wherein an oligonucleotide used as a primer for synthesizing a second strand binds to a solid phase at the 5'-side, the method comprising:
a) synthesizing the first strand cDNA using an mRNA as a template with a primer for synthesizing the first strand cDNA, and
b) synthesizing a sense strand cDNA using, as a primer for synthesizing the second strand, an oligonucleotide comprising a sequence complementary to the 3'-side of the first strand cDNA produced in a).

8. The method of claim 7, wherein the known nucleotide sequence is added to the 3'-terminal of the first strand cDNA by:
a) binding an oligonucleotide comprising a known sequence to the 5'-terminal of an mRNA, and
b) synthesizing the first strand cDNA using the mRNA of a) as a template with a primer for synthesizing the first strand.

9. The method of claim 8, wherein the oligonucleotide is bound in a) above by a method in which a CAP structure present at the 5'-terminal of the mRNA is specifically recognized.

10. A sense strand cDNA immobilized at the 5'-side, the sense strand cDNA which can be obtained by the method of any one of claims 7 to 9.

11. A method for synthesizing a cDNA library by the method of any one of claims 7 to 9 using an mRNA as a starting material.

12. A cDNA library in which sense strand cDNAs are immobilized at the 5'-side, the cDNA librarywhich can be obtained by the method of claim 11.

13. A cDNA library comprising full-length cDNAs, the cDNA library which can be obtained by the method of claim 9 using an mRNA as a starting material.

14. A secondary cDNA library which can be obtained by amplifying the cDNA library of claim 12 or 13.

15. A method for obtaining an mRNA library, the method comprising synthesizing RNAs using the cDNA library of claim 3 as a template with a DNA-dependent RNA polymerase recognizing the promoter of claim 3.

16. An mRNA library which can be obtained by the method of claim 15.

17. A method for preparing a protein library, the method comprising translating the mRNA library of claim 16 into proteins with an expression system.

18. A protein library which can be obtained by the method of claim 17.

19. A method for subtracting cDNAs, the method comprising:
a) synthesizing cDNAs used as testers,
b) hybridizing the cDNA using the sense strand cDNA library of any one of claims 1, 12, and 13 as a driver, and
c) selecting cDNAs which have or have not hybridized in b).
